# EUROPEAN PATENT SPECIFICATION

(11) **EP 3 960 106 B1**
(45) Date of publication and mention of the grant of the patent: **22.02.2023**
(21) Application number: 20803731.7
(22) Date of filing: 29.06.2020
(51) Int. Cl.: A61B 18/14, A61N 7/02, A61B 5/02, A61B 17/32, A61B 90/00, A61B 5/0215, A61B 17/22

(54) **ELECTRODE CATHETER SYSTEM**
ELEKTRODENKATHETERSYSTEM
SYSTÈME DE CATHÉTER À ÉLECTRODES

(43) Date of publication of application: 02.03.2022
(73) Proprietor: Shanghai Shape Memory Alloy Co., Ltd., Shanghai, 201612 (CN)
(72) Inventor: DING, Yishou, Beijing 102200 (CN); ZHANG, Xibo, Beijing 102200 (CN); ZHANG, Yuxin, Beijing 102200 (CN); QIAN, Jun, Beijing 102200 (CN); HUANG, Jing, Beijing 102200 (CN); PU, Zhongjie, Beijing 102200 (CN)
(74) Representative: J A Kemp LLP
(86) International application number: PCT/CN2020/098897
(87) International publication number: WO 2022/000178

(56) References cited:
- CN-A- 103 300 917
- CN-A- 103 536 352
- CN-A- 106 659 531
- US-A- 6 156 033
- US-A1- 2013 197 555
- US-A1- 2014 276 783
- US-A1- 2015 173 673
- US-A1- 2016 015 452
- US-A1- 2017 095 291
- US-A1- 2017 202 614
- US-A1- 2017 215 950

## Description

### TECHNICAL FIELD

The application relates to the technical field of interventional therapy, in particular to an electrode catheter system.

### BACKGROUND

A high incidence and prevalence of hypertension has become one of the main problems that endanger human health, and the hypertension is one of the most common chronic diseases in the world. At present, there are no accurate statistics on the prevalence of resistant hypertension, but a plurality of clinical studies have shown that patients with resistant hypertension account for 20-30% of hypertensive patients, which means that the resistant hypertension is a common chronic disease. Sympathetic nerve overexcitement has always been regarded as a basic stage in the onset of hypertension. A large number of animal experiments have proved the influence of the sympathetic nervous system on blood pressure. Clinical studies have also found that the excitement degree of the sympathetic nerve is positively correlated with the patient's blood pressure level. In the sympathetic nerve, a renal sympathetic nervous system, especially a renal sympathetic efferent nerve and a renal sympathetic afferent nerve closest to a renal artery wall, plays a decisive role in inducing and maintaining systemic hypertension.

Many clinical studies have shown that percutaneous catheter renal sympathetic radiofrequency ablation is easy to be operated and causes less complication, which can significantly and continuously lower the blood pressure of patients with refractory hypertension. It is a novel, simple and effective treatment for refractory hypertension Methods. Although radiofrequency ablation catheters provide good ideas for the treatment of refractory hypertension, and the safety and effectiveness of the treatment have been initially verified, it is necessary to screen the patient's neurological activity degree. If it is not enough, the operation cannot be used for treatment, and after the operation in the prior art, it is difficult for the ultrasonic ablation catheter system to evaluate the surgical effect of denervation before and after the operation, and it is difficult to judge whether it needs to repeat perform ablation again according to the patient's condition.

US 2017/0215950 A1 and US2016015452 A1 disclose controlled tissue ablation techniques.

### SUMMARY

The purpose of the present application is to provide an electrode catheter system to solve the technical problem in the prior art that it is difficult to evaluate the effect of surgery before or after surgery.

The invention is defined by the claims.

The beneficial effects of the electrode catheter system provided by the present application are as follows:
1. The electrode element is inserted into the renal artery blood vessel and releases an electrical signal to an inner wall of the renal artery blood vessel, and then pressure sensors are arranged in other artery blood vessels except the renal artery blood vessel to monitor a blood pressure change, and a data processing module processes the data monitored by the pressure sensor and determines the blood pressure change, and an activity degree of the nerve can be determined by measuring a blood pressure signal of the human body, so as to screen out the patients with an overactive sympathetic nerve, and a surgical effect of a denervation surgery can also be evaluated before or after the surgery, and can be used to determine whether to perform an ultrasonic ablation again.
2. A heart rate of the human body can be monitored by an electrocardiogram monitoring module, and an activity degree of the nerve can be determined by analyzing a heart rate change of human body after the electrode element is powered and a blood pressure change, which has a higher monitoring accuracy rate.
3. The electrode element is made of an elastic material, such that the control element can be controlled to contract or stretch from the outside of human body; in a normal state, the electrode element is received in the interventional catheter, after being arranged in the human body, the electrode element can be elastically fitted to the inner wall of the blood vessel to adapt to the blood vessel size, a control method of which is simple, and can be brought into human body by the interventional catheter and leads to less damage to human body.
4. An ablation element is arranged at a front end of the interventional catheter, which can combine ablation and measurement, and only one interventional catheter is required to achieve preoperative screening, ultrasonic ablation and postoperative measurement, and the ultrasonic ablation can be performed continuously after measuring an unsuccessful denervation surgery, which is convenient to use and can cause less damage to human body.

### BRIEF DESCRIPTION OF THE DRAWINGS

In order to illustrate the embodiments of the present application or the technical solutions in the prior art more clearly, the drawings used in the embodiments or the description of the prior art will be briefly described below. Obviously, the drawings attached in the following description only represent some examples of the present application, and those skilled in the art can obtain other drawings based on these drawings without any creative intellectual work.
Figure 1 is a schematic structural view showing the electrode catheter system in Example 1 of the present application, in which the electrode element extends out of the intervention catheter.
Figure 2 is a schematic structural view showing the electrode catheter system in Example 1 of the present application, in which the electrode element is received in the interventional catheter.
Figure 3 is a schematic structural view showing the interventional catheter in Example 1 of the present application.
Figure 4 is a partial enlarged cross-sectional view showing part A in Figure 3.
Figure 5 is a sectional structural view in B-B direction in Figure 3.
Figure 6 is a schematic structural view showing the interventional catheter in Example 2 of the present application.

### Reference Signs:

1. interventional catheter; 2. pressure sensor; 3. electrocardiogram monitor; 4. handle; 5. renal artery blood vessel; 11. ablation element; 12. electrode element; 13. first filament guide cavity; 14. second filament guide cavity; 15. water inlet cavity; 16. water outlet cavity; 121. control filament; 111. balloon; 112. ultrasonic transducer; 41. control button;

### DETAILED DESCRIPTION

The present application will be further described in detail below with reference to the drawings and examples. Obviously, the described embodiments represent part of, but not all of the embodiments of the present application. Based on the embodiments of the present application, all other embodiments obtained by a person skilled in the art without creative work shall fall within the scope of the present application.

In the description of the present application, it should be understood that the terms "center", "upper", "lower", "left", "right", "vertical", "horizontal", "inside" and "outside" indicate orientation or positional relationship which are based on the orientation or positional relationship shown in the drawings, and it is merely for convenience of describing the present application and simplifying the description, rather than indicating or implying that the indicated device or element must have a particular orientation, and be constructed and operated in a particular orientation. Therefore these terms should not be understood as limiting the scope of the present application. In addition, the terms "first", "second", and "third" are only used for descriptive purposes, and cannot be understood as indicating or implying relative importance.

In the description of the present application, unless otherwise explicitly stated or specified, it should be noted that the terms "disposed", "connected" and "connecting" should be understood in a broad sense. For example, a connection can be a fixed or detachable connection, or an integral connection; a mechanical connection or an electrical connection; a direct or indirect connection through an intermediate medium, and it can be an internal communication between two elements. For a person skilled in the art, the specific meaning of the above-mentioned term in the present application can be understood in specific situation.

In the present application, "proximal end" and "distal end" refer to a relative orientation, relative position and relative direction of the elements or actions among each other from the perspective of the doctor using a medical device, although "proximal end" and "distal end" are not restrictive, and the "proximal end" usually refers to an end of the medical device that is close to the doctor during normal operation, and the "distal end" usually refers to the end that firstly enters the human body of the patient.

In addition, the technical features involved in the different embodiments of the present application described below can be combined with each other as long as they do not conflict with each other.

### Example 1

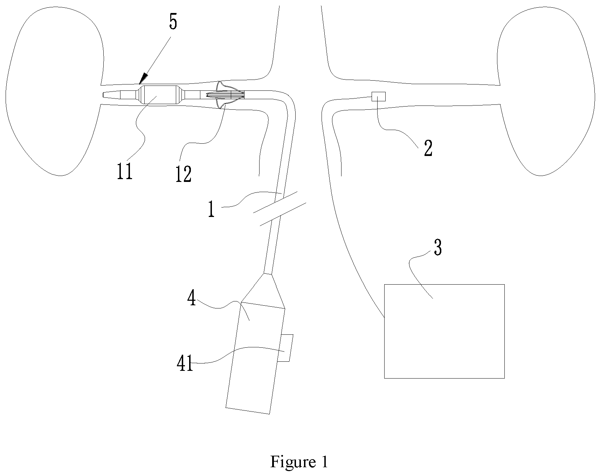

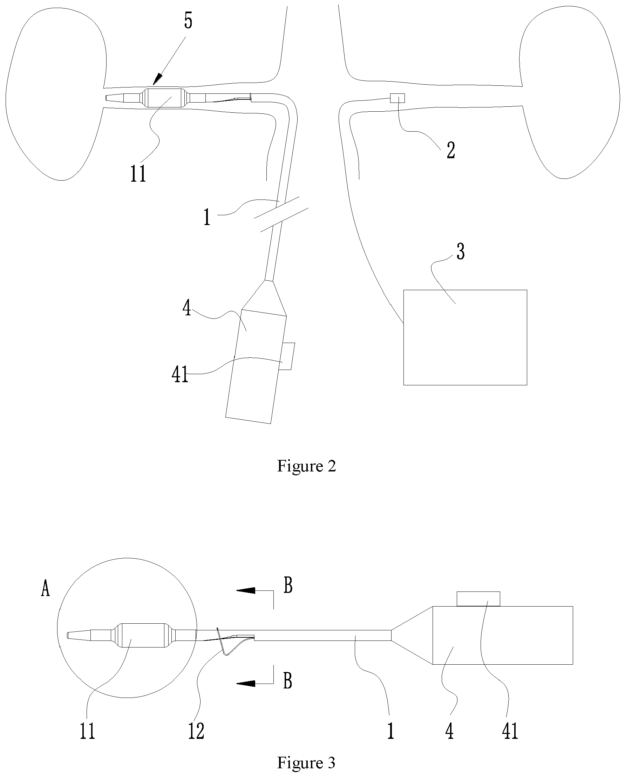

As shown in Figure 1 and Figure 2, the electrode catheter system of the present application will be described herein. The electrode catheter system includes an interventional catheter 1, a pressure sensor 2 and a data processing module (not shown). The interventional catheter 1 can be inserted into one end of a renal artery 5, and the interventional catheter 1 is provided with an ablation element 11 at a distal end thereof. The ablation element 11 is provided with an electrode element 12 at a proximal end thereof, the electrode element 12 can release an electrical signal towards an inner wall of the renal artery blood vessel 5. The pressure sensor 2 located in the other arterial vessels 5 can measure the pressure in the blood vessel in real time, that is to measure blood pressure, and the measured pressure in the blood vessel is converted into blood pressure through the data processing module, such that the nerve activity degree can be determined according to the blood pressure.

The data processing module can be a data processor of a conventional blood pressure instrument in the prior art, which can convert a value of the pressure sensor 2 disposed in the blood vessel into a blood pressure value of human body, and the data processing module can directly determine whether the blood pressure exceeds a certain threshold value to determine whether the nerve activity degree is too high, or the blood pressure change value can be directly fed back to a screen, and the operator can conclude whether the nerve activity degree is too high in view of the blood pressure change rule, which are not exclusively limited herein.

The pressure sensors 2 is arranged in other blood vessel except the renal artery blood vessel 5 that is provided with the interventional catheter, so as to prevent the electrode signal of the electrode element 12 from interfering with the measurement. The pressure sensor 2 is located in the other renal artery blood vessel, which is beneficial for easy operation during surgery and the measurement result can be more accurate.

Furthermore, in order to further enhance measurement accuracy of nerve activity degree, an electrocardiogram monitoring module 3 can be added, and a heart rate change monitored by the electrocardiogram monitoring module 3 can be used to judge the nerve activity degree. The electrocardiogram monitoring module 3 can be a conventional electrocardiogram monitoring module 3 used in the prior art. The electrocardiogram monitoring module 3 is in signal communication with the data processing module, and the heart rate measured by the electrocardiogram monitoring module 3 can be received through the data processing module. The data processing module can directly judge whether the heart rate exceeds a certain threshold, thereby making a conclusion whether the nerve activity degree is too high, or directly feedback a heart rate change value on a screen, and the operator judges whether the nerve activity degree is too high based on a change rule of the heart rate and blood pressure, which is not exclusively limited herein.

The specific judging standard for nerve activity degree belongs to existing technology. The nerve activity degree can be judged by measuring the blood pressure signal of human body, and a heart rate and other information can be used for auxiliary judgment, so as to screen out patients with overactive sympathetic nerves. A surgical effect of denervation surgery before or after the operation can be evaluated so as to decide whether to perform ultrasonic ablation again.

The operation steps for neuromapping before the surgery are as follows:
S1. the electrode element 12 releases an electrical signal towards the renal artery blood vessel 5 at one side;
S2. the electrocardiogram monitor 3 monitors a heart rate, and the pressure sensor 2 monitors a blood pressure change in the renal artery vein 5 at the other side. The nerve activity degree of the patient can be judged by judging whether the heart rate and blood pressure changes reach a certain threshold;
If the patient's heart rate and blood pressure change are lower than the threshold, it can be determined that the patient's nerve activity degree is insufficient, and a hypertension due to sympathetic nerve overexcitation can be ruled out, and other treatments may be required;
If the patient's heart rate and blood pressure change reach the threshold, the patient's nerve activity degree is relatively strong, which may be due to hypertension caused by sympathetic nerve overexcitation, and an ultrasonic ablation is required to treat the patient.

The operation steps for neuromapping to measure the recovery state of the patient after the operation are as follows:
S 1. the electrode element 12 release an electrical signal towards the renal artery blood vessel 5 at one side;
S2. the heart rate is monitored by the electrocardiogram monitor 3, and the pressure sensor 2 is used to monitor the blood pressure change in the renal artery blood vessel 5 on the other side, and the patient's nerve activity degree is judged by judging whether the heart rate and blood pressure change reaches the threshold;
If the patient's heart rate and blood pressure change are lower than the threshold, the patient's nerve activity degree has been reduced to the standard value, which proves that the effect of the operation is well, and the treatment can be ended;
If the patient's heart rate and blood pressure changes still reach the threshold, the patient's nerve activity degree is still strong, it can be determined that the denervation surgery has not fully achieved the effect, or an ultrasonic ablation location may be required to be adjusted, and the ultrasonic ablation should be repeated until the patient's heart rate and blood pressure change are below the threshold.

Through the above methods, the surgical effect of denervation surgery can be evaluated before or after the operation, and it can be determined whether or not to perform ultrasonic ablation again. The pressure sensor 2 can be a pressure sensor 2 in the prior art that can be arranged in human blood vessel to measure intravascular pressure, and the electrocardiogram monitor 3 can also be a conventional electrocardiogram monitoring device in the prior art. The pressure sensor 2 and the electrocardiogram monitor 3 both can be connected to a controller together, and the measured results can be processed and analyzed uniformly through the controller, the measured values can also be displayed separately, and the operator can make a judgment according to the measured value, which is not limited herein.

Further, as shown in Figures 3 to 5, as a specific embodiment of the electrode catheter system of the present application, the electrode element 12 is made of an elastic metal material, and the electrode element 12 can be driven by the control filament 121 to be received in the interventional catheter 1, or to be in close contact with the inner wall of the renal artery blood vessel 5. The control filament 121 is arranged in the interventional catheter 1, and the interventional catheter 1 is a multi-cavity catheter. The interventional catheter 1 is provided with a first filament guide cavity 13 for receiving the control filament 121. The control filament 121 can slide in the first filament guide cavity 13. Certainly, according to actual conditions and specific requirements, in other embodiments of the present application, the electrode element 12 may be made of other elastic and conductive materials, such as conductive foam, etc., which is not exclusively limited here.

A handle 4 is located at the proximal end of the interventional catheter 1, which is convenient for the operator to hold and control the catheter 1. The handle 4 is provided with a control button 41, which can be used to control the stretch and contraction of the control filament 121. One end of the control filament 121 enters the renal artery blood vessel 5 through the first filament guide cavity 13, and the other end of the control filament 121 can be connected to the outer handle 4 via the interventional catheter 1 to control the control filament 121 from the outside, and then control the retracting and stretching of the electrode element 12. The control button 41 can not only control the control filament 121 to move along a length direction of the interventional catheter 1, but also control the control filament 121 to rotate along a circumferential direction of the interventional catheter 1.

For the convenience of the operator, the handle 4 can be provided with other control buttons (not shown in the figure), such as a control button that controls power on and off of the electrode element 12 or the ablation element 11. The control button can be connected to a wire and control the power on and off of the wire.

As a specific embodiment of the electrode catheter system of the present application, an electrode wire is generally used as the electrode element 12, and the electrode wire is preformed to have an arc shape or circular ring shape. Two ends of the electrode wire are free ends, and both ends are directly fixedly connected to or integrally formed with the control filament 121. The electrode wire can move along the length direction of the interventional catheter 1 under the driving of the control filament 121, so that the electrode wire can move in the interventional catheter 1. Before entering to a predetermined position, the electrode element 12 is compressed and received in the interventional catheter 1 to avoid additional damage; after entering to the predetermined position, the control filament 121 drives the electrode element 12 to move toward the distal end, at this time the electrode element 12 can be opened and gradually abuts against the inner wall of the blood vessel. After the operation is completed, the control filament 121 drives the electrode element 12 to move toward the proximal end. At this time, the electrode element 12 can be retracted and received in the interventional catheter 1. Certainly, according to actual conditions and specific requirement, in other embodiments of the present application, the electrode element 12 can also be preformed into a square or triangle, etc., which is not exclusively limited herein.

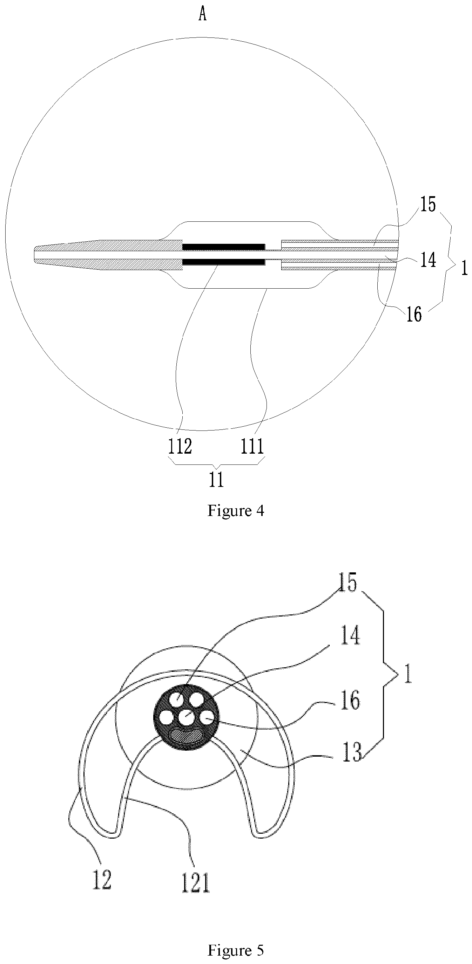

Further, please refer to Figure 4, as a specific embodiment of the electrode catheter system of the present application, the interventional catheter 1 is also provided with an ablation element 11, and the ablation element 11 represents a device that can perform ultrasonic ablation during an ablation surgery, and the ablation element 11 is located at the distal end of the interventional catheter 1, the electrode element 12 is located at the proximal end of one side near the ablation element 11, and the electrode element 12 and the ablation element 11 are both located in the renal artery blood vessel 5.

The ablation element 11 comprises a balloon 111 and an ultrasonic transducer 112 disposed in the balloon 111, the balloon 111 is sleeved on the interventional catheter 1 and fixed to the distal end of the interventional catheter 1. The balloon 111 can be inflated after injection of liquid or gas, and can be wrapped around the exterior of the ultrasonic transducer 112, and protect the ultrasonic transducer 112 to avoid direct contact with the human body. The ultrasonic transducer 112 can be a conventional ultrasonic transducer 112 in the prior art capable of realizing ultrasonic ablation, and one or more ultrasonic transducers 112 can be provided as required.

The interventional catheter 1 is a multi-cavity catheter. The interventional catheter 1 comprise a plurality of cavities therein comprising a first filament guide cavity 13, a second filament guide cavity 14, a water inlet cavity 15 and a water outlet cavity 16. The first filament guide cavity 13 is configured for the control filament 121 to pass therethrough, and the length thereof is extended from the handle 4 to the control filament 121; the second filament guide cavity 14 is configured for other operation guide wires to pass therethrough, and the second filament guide cavity 14 generally penetrates through the entire interventional catheter 1, and both the water inlet cavity 15 and the water outlet cavity 16 are communicated to the balloon 111, and the water inlet cavity 15 and the water outlet cavity 16 are supplied with cold water to pass through, and the proximal end of the water inlet cavity 15 is connected with a pressurized syringe which contains cold water. The distal end of the water inlet cavity 15 is in communication with the balloon 111, and a plurality of water outlets are arranged on a side wall of the distal end, and the water outlets are in communication with the balloon 111. When an ultrasonic ablation is required, the balloon 111 is filled with cold water therein, at the same time, cold water can flow out from the water outlet cavity 16 to the outside of human body, such that the cold water in the balloon 111 can be circulated, and when the ultrasonic ablation is completed, the cold water can be completely discharged from the water outlet cavity 16, which can effectively prevent the temperature of ultrasonic transducer 112 being too high during operation.

The interventional catheter 1 is also provided with a wire (not shown), and the wire can supply power to the ultrasonic transducer 112 and the electrode element 12, and the wire can be directly located in the interventional catheter 1, or can be received in the second guide filament cavity 14 or other cavities.

Preferably, in order to facilitate the wire to provide power to the ultrasonic transducer 112, an inner distal end of the balloon 111 only comprises the second filament guide cavity 14, and an outer edge of the second filament guide cavity 14 is directly provided a transducer base (not shown in the figure) configured to fix the ultrasonic transducer 112. The wire is arranged to pass through the interior of the second filament guide cavity 14 and connected to the ultrasonic transducer 112 through the transducer base. The transducer base can also be a conventional transducer base in the prior art.

During use, the interventional catheter 1 is arranged into the patient's renal artery 5 and a blood pressure change and heart rate in the renal artery blood vessel 5 on the other side are monitored. After the interventional catheter 1 is arranged at a specific position in the renal artery blood vessel 5, the control button controls the control filament 121 to move along the length direction of the interventional catheter 1, and the control filament 121 drives the electrode element 12 to move towards the distal end. The electrode element 12 is released and expanded under an elasticity action until the electrode element 12 abuts on an inner wall of the renal artery blood vessel 5. The electrode element 12 is controlled to release an electrical signal, and if a condition of an ultrasonic ablation is met, water is injected into the balloon 111 through a water inlet and returned through a water outlet to realize water circulation, and the ultrasonic transducer 112 starts to perform ultrasonic ablation.

If the patient's nerve activity degree is measured to decrease to a standard value, the ultrasonic ablation is completed, the ultrasonic transducer 112 is stopped to work and the water injection into the balloon 111 is stopped, the water in the balloon 111 flows out from the water outlet. The control filament 121 drives the electrode element 12 to move toward the proximal end, and the electrode element 12 is compressed under the action of the inner wall of the interventional catheter 1 and abuts against the inner wall of the interventional catheter 1 under the elastic ability thereof.

### Example 2

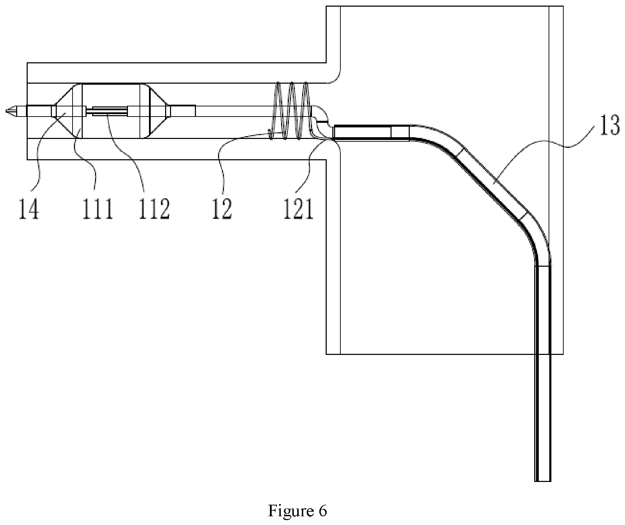

As shown in Figure 6, as another specific embodiment of the electrode catheter system of the present application, the difference between Embodiment 2 and Embodiment 1 is that the electrode element 12 is preformed to have a spiral ring shape, and one end of the electrode element 12 is fixedly connected to the control filament 121. The electrode element 12 is still an electrode wire, which is preformed to have a spiral ring shape. The electrode wire also comprises two free ends, one of which is directly fixedly connected to or is integrally formed with the control filament 121, and the other free end is located at a distal end of the control filament 121. The control filament 121 can be moved along a length direction of the interventional catheter 1 in the first filament guide cavity 13 and can rotate around a circumference of the interventional catheter 1 to gradually release the electrode wire to the outside of the catheter 1 to abut against an inner wall of the renal artery blood vessel 1, at this time, the electrode wire is in contact with the inner wall of the renal artery blood vessel 1 with larger abutment area and range, so that when the electrode element 12 releases an electrical signal toward the renal artery blood vessel 5, the area to be stimulated is wider. Certainly, according to actual condition and specific requirement, in other examples of the present application, the electrode element 12 may also have a spiral ellipse shape, a spiral triangle shape, etc., which is not exclusively limited herein.

Certainly, the above-mentioned embodiments are only examples for clear description, and are not intended to restrict the embodiment. For an ordinary person skilled in the art, other changes or modifications in different forms can be made on the basis of the above description, the scope of the present invention only limited by the scope of appended claims.

## Claims

1. An electrode catheter system, comprising
an interventional catheter (1), provided with an electrode element (12) that can release an electrical signal toward an inner wall of a renal artery blood vessel (5);
a pressure sensor (2) for intervening to an artery blood vessel; and
a data processing module, connected with the pressure sensor (2),
wherein,
the pressure sensor (2) is configured to be arranged in the other renal artery blood vessel;
a control filament (121) and a first filament guide cavity (13) that receives the control filament (121) are arranged in the interventional catheter (1), and the electrode element (12) is connected with the control filament (121), and the control filament (121) slides in the first filament guide cavity (13) by an external force,.
the interventional catheter (1) is further provided with an ablation element (11), and the ablation element (11) is located at a distal end of the interventional catheter (1), **characterized in that** the
electrode element (12) is located at a proximal end of the ablation element (11),
wherein the ablation element (11) further comprises a balloon (111), fixed to the distal end of the interventional catheter (1); and
an ultrasonic transducer (112) disposed in the balloon (111).

2. The electrode catheter system according to claim 1, further comprising
an electrocardiogram monitoring module (3), connected with the data processing module and configured to monitor heartbeat.

3. The electrode catheter system according to claim 1, wherein
the electrode element (12) is made of an elastic metal material, and the electrode element (12) has a first state and a second state, wherein
the first state in which the electrode element (12) is received in the intervention catheter (1), and
the second state in which the electrode element (12) is fitted with the inner wall of the renal artery blood vessel (5).

4. The electrode catheter system according to claim 1, wherein
the electrode element (12) is preformed to have an arc shape, and both ends of the electrode element (12) are fixedly connected to the control filament (121).

5. The electrode catheter system according to claim 1, wherein
the electrode element (12) is preformed to have a spiral ring shape, and one end of the electrode element (12) is fixedly connected with the control filament (121).

6. The electrode catheter system according to claim 1, wherein
a second filament guide cavity (14) and a wire are arranged in the intervention catheter (1), and the second filament guide cavity (14) is disposed in the balloon (111), and a transducer base is located at an outer edge of the second filament guide cavity (14), and the ultrasonic transducer (112) is fixed on the transducer base, and the ultrasonic transducer (112) is connected to the wire.

7. The electrode catheter system according to claim 1, wherein
a water inlet cavity (15) and a water outlet cavity (16) are arranged in the interventional catheter (1), and both of the water inlet cavity (15) and the water outlet cavity (16) are communicated with the balloon (111), respectively.

8. The electrode catheter system according to any one of claims 1 to 5, wherein
a proximal end of the interventional catheter (1) is provided with a handle (4), and the handle (4) is provided with a control button (41) to control the electrode element (12).

## Patentansprüche

1. Elektrodenkathetersystem, das Folgendes umfasst:
einen Interventionskatheter (1), der mit einem Elektrodenelement (12) versehen ist, das ein elektrisches Signal an eine Innenwand eines Nierenarterienblutgefäßes (5) abgeben kann;
einen Drucksensor (2) zum Eingreifen in ein Arterienblutgefäß; und
ein Datenverarbeitungsmodul, das mit dem Drucksensor (2) verbunden ist, wobei,
der Drucksensor (2) dafür konfiguriert ist, in dem anderen Nierenarterienblutgefäß angeordnet zu werden;
ein Steuerfilament (121) und ein erster Filamentführungshohlraum (13), der das Steuerfilament (121) aufnimmt, in dem Interventionskatheter (1) angeordnet sind, und das Elektrodenelement (12) mit dem Steuerfilament (121) verbunden ist, und der Steuerfilament (121) durch eine äußere Kraft in dem ersten Filamentführungshohlraum (13) gleitet,
der Interventionskatheter (1) ferner mit einem Ablationselement (11) versehen ist und das Ablationselement (11) an einem distalen Ende des Interventionskatheters (1) angeordnet ist, **dadurch gekennzeichnet, dass** das Elektrodenelement (12) an einem proximalen Ende des Ablationselements (11) angeordnet ist, wobei das Ablationselement (11) ferner Folgendes umfasst:
einen Ballon (111), der an dem distalen Ende des Interventionskatheters (1) befestigt ist; und
einen Ultraschallwandler (112), der in dem Ballon (111) angeordnet ist.

2. Elektrodenkathetersystem nach Anspruch 1, das ferner Folgendes umfasst:
ein Elektrokardiogramm-Überwachungsmodul (3), das mit dem Datenverarbeitungsmodul verbunden und zur Überwachung des Herzschlags konfiguriert ist.

3. Elektrodenkathetersystem nach Anspruch 1, wobei
das Elektrodenelement (12) aus einem elastischen Metallmaterial hergestellt ist und das Elektrodenelement (12) einen ersten Zustand und einen zweiten Zustand aufweist, wobei
in dem ersten Zustand das Elektrodenelement (12) in dem Interventionskatheter (1) aufgenommen ist, und
in dem zweiten Zustand das Elektrodenelement (12) an der Innenwand des Nierenarterienblutgefäßes (5) angebracht ist.

4. Elektrodenkathetersystem nach Anspruch 1, wobei
das Elektrodenelement (12) bogenförmig vorgeformt ist und beide Enden des Elektrodenelements (12) fest mit dem Steuerfaden (121) verbunden sind.

5. Elektrodenkathetersystem nach Anspruch 1, wobei
das Elektrodenelement (12) so vorgeformt ist, dass es eine Spiralringform aufweist und ein Ende des Elektrodenelements (12) fest mit dem Steuerfaden (121) verbunden ist.

6. Elektrodenkathetersystem nach Anspruch 1, wobei
in dem Interventionskatheter (1) ein zweiter Filamentführungshohlraum (14) und ein Draht angeordnet sind, und der zweite Filamentführungshohlraum (14) in dem Ballon (111) angeordnet ist, und eine Wandlerbasis an einem äußeren Rand des zweiten Filamentführungshohlraums (14) angeordnet ist, und der Ultraschallwandler (112) an der Wandlerbasis befestigt ist, und der Ultraschallwandler (112) mit dem Draht verbunden ist.

7. Elektrodenkathetersystem nach Anspruch 1, wobei
ein Wassereinlasshohlraum (15) und ein Wasserauslasshohlraum (16) in dem Interventionskatheter (1) angeordnet sind und sowohl der Wassereinlasshohlraum (15) als auch der Wasserauslasshohlraum (16) jeweils mit dem Ballon (111) in Verbindung stehen.

8. Elektrodenkathetersystem nach einem der Ansprüche 1 bis 5, wobei
ein proximales Ende des Interventionskatheters (1) mit einem Griff (4) versehen ist und der Griff (4) mit einem Steuerknopf (41) versehen ist, um das Elektrodenelement (12) zu steuern.

## Revendications

1. Système de cathéter à électrodes, comprenant
un cathéter interventionnel (1), pourvu d'un élément d'électrode (12) qui peut libérer un signal électrique vers une paroi interne d'un vaisseau sanguin de l'artère rénale (5) ;
un capteur de pression (2) pour intervenir sur un vaisseau sanguin artériel ; et
un module de traitement de données, connecté au capteur de pression (2),
dans lequel
le capteur de pression (2) est configuré pour être agencé dans l'autre vaisseau sanguin de l'artère rénale ;
un filament de commande (121) et une première cavité de guidage de filament (13) qui reçoit le filament de commande (121) sont agencés dans le cathéter d'intervention (1), et l'élément d'électrode (12) est relié au filament de commande (121), et le filament de commande (121) coulisse dans la première cavité de guidage de filament (13) par une force extérieure,
le cathéter d'intervention (1) est en outre pourvu d'un élément d'ablation (11), et l'élément d'ablation (11) est situé à une extrémité distale du cathéter d'intervention (1), **caractérisé en ce que** l'élément d'électrode (12) est situé à une extrémité proximale de l'élément d'ablation (11), dans lequel l'élément d'ablation (11) comprend en outre
un ballonnet (111), fixé à l'extrémité distale du cathéter d'intervention (1) ; et
un transducteur ultrasonique (112) disposé dans le ballonnet (111).

2. Système de cathéter à électrodes selon la revendication 1, comprenant en outre un module de surveillance d'électrocardiogramme (3), connecté au module de traitement de données et configuré pour surveiller le rythme cardiaque.

3. Système de cathéter à électrodes selon la revendication 1, dans lequel
l'élément d'électrode (12) est constitué d'un matériau métallique élastique, et l'élément d'électrode (12) a un premier état et un deuxième état, dans lequel
le premier état dans lequel l'élément d'électrode (12) est reçu dans le cathéter d'intervention (1), et
le deuxième état dans lequel l'élément d'électrode (12) est ajusté avec la paroi interne du vaisseau sanguin de l'artère rénale (5).

4. Système de cathéter à électrodes selon la revendication 1, dans lequel
l'élément d'électrode (12) est préformé pour avoir une forme d'arc, et les deux extrémités de l'élément d'électrode (12) sont connectées de manière fixe au filament de commande (121).

5. Système de cathéter à électrodes selon la revendication 1, dans lequel
l'élément d'électrode (12) est préformé pour avoir une forme d'anneau en spirale, et une extrémité de l'élément d'électrode (12) est reliée de manière fixe au filament de commande (121).

6. Système de cathéter à électrodes selon la revendication 1, dans lequel
une deuxième cavité de guidage de filament (14) et un fil sont disposés dans le cathéter d'intervention (1), et la deuxième cavité de guidage de filament (14) est disposée dans le ballonnet (111), et une base de transducteur est située au niveau d'un bord extérieur de la deuxième cavité de guidage de filament (14), et le transducteur ultrasonique (112) est fixé sur la base de transducteur, et le transducteur ultrasonique (112) est connecté au fil.

7. Système de cathéter à électrode selon la revendication 1, dans lequel
une cavité d'entrée d'eau (15) et une cavité de sortie d'eau (16) sont agencées dans le cathéter d'intervention (1), et à la fois la cavité d'entrée d'eau (15) et la cavité de sortie d'eau (16) communiquent avec le ballonnet (111), respectivement.

8. Système de cathéter à électrodes selon l'une quelconque des revendications 1 à 5, dans lequel
une extrémité proximale du cathéter interventionnel (1) est pourvue d'une poignée (4), et la poignée (4) est pourvue d'un bouton de commande (41) pour commander l'élément d'électrode (12).
